# EUROPEAN PATENT APPLICATION

(11) **EP 4 810 488 A1**
(43) Date of publication of application: **23.09.2026**
(21) Application number: 25165159.2
(22) Date of filing: 20.03.2025
(51) Int. Cl.: C07D 303/23, C07D 303/36, C07C 323/31

(54) **COMPOUNDS FOR THE FORMATION OF VITRIMERS**

(71) Applicant: Airbus SAS, 31700 Blagnac Cedex (FR)
(72) Inventor: Beier, Uwe, 82024 Taufkirchen (DE); Hübner, Fabian, 82024 Taufkirchen (DE); Carré, Camille, 82024 Taufkirchen (DE); Meer, Thomas, 82024 Taufkirchen (DE); Machunze, Wolfgang, 82024 Taufkirchen (DE)
(74) Representative: Isarpatent

(57) **Abstract**

The present invention relates to a compound, a composition comprising one or more of such compounds, a vitrimer produced using one or more of such compounds, the use of such a vitrimer, as well as a method of manufacturing a vitrimer.

## Description

### TECHNICAL FIELD OF THE INVENTION

The present invention relates to a compound, a composition comprising one or more of such compounds, a vitrimer produced using one or more of such compounds, the use of such a vitrimer, as well as a method of manufacturing a vitrimer.

### TECHNICAL BACKGROUND

In aviation, lightweight construction is a crucial element. Minimized structural weight is intrinsic to aviation, enabling challenging missions but also reducing the operator cost by saving fuel and finally contributing to sustainability targets. Composites are nowadays a key facilitator of optimized aircraft weight. However, there are some limitations and drawbacks of current composite technologies, namely high cost, low rate capability, insufficient composite joining technologies and last but not least the relatively high environmental footprint of a composite part itself. The environmental footprint of composites consists of the efforts to produce the raw material, the subsequent manufacturing and recycling processes. The latter can be considered to enable a credit for a second life cycle.

Composite materials for all kinds of aerospace applications are made with reinforcement fibers and a polymeric matrix. The polymeric matrix is either thermoset (TS) or thermoplastic (TP) with both advantages and disadvantages in terms of processing, thermal/mechanical properties, bonding/fusing and recycling. The two polymeric materials differ in their chemical nature.

Thermosets yield highest laminar and interlaminar quality of composites, which is required for structural applications. But the environmental footprint is high due to the extensive curing and consolidation steps, which are usually highly energy demanding, e.g. based on autoclave processes. When manufactured to final shape, bonding of thermoset is complex, time and cost demanding due to its insoluble, non-meltable and non-malleable chemical nature. Recycling approaches are non-satisfactory to date, because it requires breaking covalent chemical bonds to recover the monomers, with high energy cost.

Thermoplasts can be recycled comparably easily. However, for high laminar and interlaminar quality, high temperatures are necessary for consolidation. Additionally, the environmental footprint of high performance thermoplastic processing is amongst the highest, particularly also in view of the necessary very high temperature regimes for consolidation. In order to eliminate that energy intensive step, research on in-situ consolidation during automated fiber placement (AFP) processes has been conducted. Due to material specific limitations, particularly diffusion speed of the long chain molecules, the quality achieved is not on an appropriate level required for aviation.

The described processes to manufacture thermoset- and thermoplastic-based composites are not only energetically demanding, but are also time consuming. If highest qualities are required, again autoclave-manufacturing is the process of choice. Additionally, a demand for extensive hand labor for the autoclave bagging occurs.

Today's material and manufacturing processes, for both thermoplastic (TP) and thermoset (TS), get optimized using various technologies. However, detailed studies show that even in a long term perspective there are some limitations, hindering a close to zero environmental footprint.

The special nature of fiber composites are strongly benefiting from specific designs to enable lightweight construction, thereby exploiting the light-weight potential to the next level. Particularly, joining designs are benefitting from shear load designs, which are enabled by welding or adhesive bonding. Both are appropriate for thin-walled structures and a contributor to lightweight design compared to mechanical joining, but are also time / cost consuming and more importantly challenging regarding certification rules in aviation.

In the current literature the polymeric material class "vitrimers", characterized by their reversible covalent bonding networks, e.g. comprising disulfide bonds, are seen as potential enablers of easily recyclable and repairable composites. They can be seen as potential enablers for joining, bonding and thus repair and recycling to target end-of-life issues, i.e. forming the basis for easily recyclable and repairable composites. An example of vitrimers can e.g. be found in US 11713370 B1.

However, vitrimers and uncured mixtures for forming such vitrimers described in literature still lack properties such as proper manufacturability, thermal stability, sustainable raw material production and performance in cryogenic environments. Vitrimers chemistry as identified in literature and IP research are in its infancy and molecules are rather investigated because they are easy to access and not because they represent the very edge of performance characteristics anticipated and needed for their exploitation. This is true for most of the vitrimer classes and is particularly true for disulfide-based vitrimers. Most of the time they appear to be clones of currently used epoxy resins or amine hardeners. Imine bondings can achieve vitrimeric polymer properties as well, but with different temperature/mechanical performance profiles.

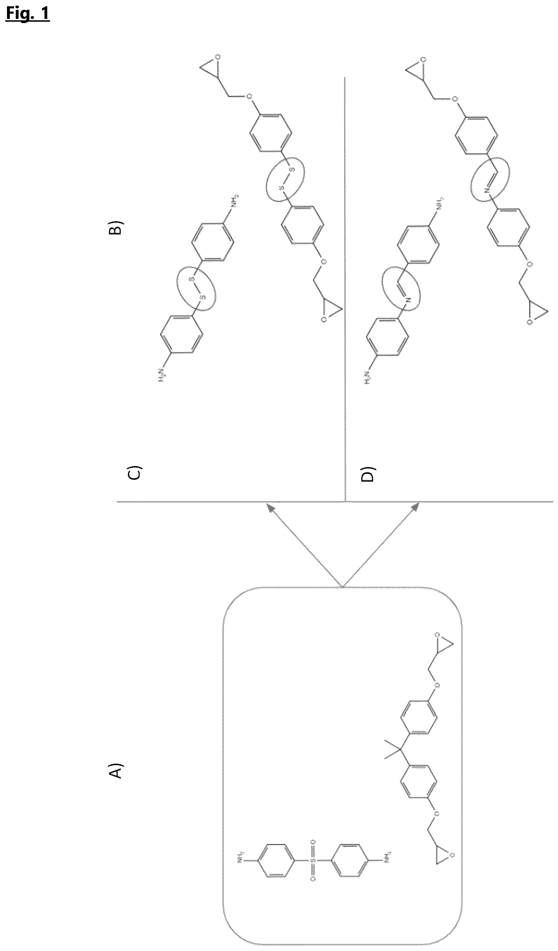

Vitrimer derivatives such as those shown in Fig. 1 work in principle, but the opportunity of improving/tailoring the tack of uncured mixtures, vitrimer functionalities, ease of recycling, tailored curing reactivities, viscosities, performance in cryogenic environments, enthalpies, etc. would certainly unlock a wide range of applications.

For special applications, in particular in aviation, it is desirable to provide uncured mixtures with specially adapted properties. Such properties include improved tack, elasticity and viscosity. It is further desirable to provide vitrimers with improved performance in cryogenic environments. Such uncured mixtures and vitrimers with the aforementioned properties have not yet been developed.

### SUMMARY OF THE INVENTION

In order to overcome the above drawbacks, the present invention aims to improve the properties of disulfide and imine vitrimers and uncured mixtures for forming such vitrimers by using engineered molecules. Thereby key properties such as prepreg tack, elasticity and performance in cryogenic environments can be brought to a new level at the same time. These inventors found that vitrimers formed using the identified compounds as well as uncured mixtures containing said identified compounds unlock various applications while leading to the following benefits:
- reliable & certifiable bonding
- debonding on demand
- manufacturing cost reduction
- high rate production capability
- reduced environmental footprint
- improved tack and elasticity
- improved toughness performance in cryogenic environments

An aspect of the invention relates to a compound according to Formula (I) as shown in claim 1 as appended.

Further disclosed is a composition, comprising one or more of the compounds according to the above aspect.

Also disclosed is a method of manufacturing a polymeric network and/or vitrimer. The method comprises providing at least one compound according to the above aspect or the composition as disclosed above, and polymerizing, co-polymerizing and/or cross-linking the one or more compound or the composition to obtain a polymeric network and/or vitrimer.

Further disclosed is a vitrimer or composite, produced using one or more of the compounds of the above aspect and/or the composition as disclosed above. Additionally, the invention relates to the use of such a vitrimer as adhesive, matrix resin or for surface functionalization, in particular in aircraft composites.

Further aspects and embodiments of the invention are disclosed in the dependent claims and can be taken from the following description, figures and examples, without being limited thereto.

### BRIEF SUMMARY OF THE DRAWINGS

The present invention is explained in more detail below with reference to the embodiments shown in the schematic figures:
- Fig. 1: exemplary monomers for the formation of thermosets (left) and vitrimers (right) according to the state of the art. A) classic TS, B) vitrimers, C) disulfide, D) imine.

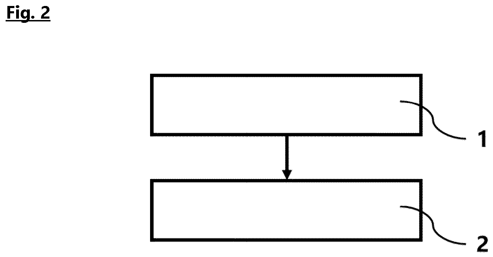

- Fig. 2: schematic representation of a method according to the invention, comprising the steps of providing at least one compound according to the first aspect of the invention or a composition according to the second aspect of the invention 1, and polymerizing, co-polymerizing, cross-linking and/or curing the at least one compound or the composition to obtain the polymeric network and/or vitrimer 2.

In the figures of the drawing, elements, features and components which are identical, functionally identical and of identical action are denoted in each case by the same reference designations unless stated otherwise.

### DESCRIPTION OF EXEMPLARY EMBODIMENTS

### Definitions

Unless defined otherwise, technical and scientific terms used herein have the same meaning as commonly understood by one of ordinary skill in the art to which this invention belongs.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a", "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise.

As used herein, the terms "comprises", "comprising", "contains", "containing", "includes", "including", "has", "having" or any other variation thereof, are intended to cover a non-exclusive inclusion. For example, a process, method, article, or apparatus that comprises a list of elements is not necessarily limited to only those elements, but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

The use of the term "at least one" or "one or more" will be understood to include one as well as any quantity more than one. In addition, the use of the phrase "at least one of X, Y, and Z" will be understood to include X alone, Y alone, and Z alone, as well as any combination of X, Y, and Z.

The use of ordinal number terminology (i.e., "first", "second", "third", "fourth", etc.) is solely for the purpose of differentiating between two or more items and, unless explicitly stated otherwise, is not meant to imply any sequence or order or importance to one item over another or any order of addition.

As used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

As used herein, 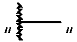 and 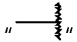 mean that the group or compound shown is linked to the neighboring group or compound of the respective structure via a covalent bond. For Z of Formula (I), for example, this means that the compounds or groups shown for Z are bound to L₁ or A₁ at the position labelled with the corresponding symbol. If a bond crosses or is "drawn over" another bond of a ring shown, such as 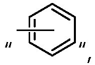 this means that the bond in question can be at any possible position of the ring.

In a first aspect, the present invention relates to a compound according to Formula (I):

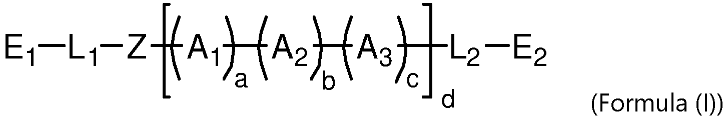

In Formula (I), A₁ is selected from the group consisting of 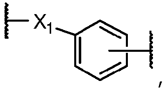 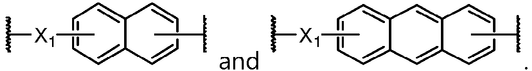 In the groups shown for A₁, 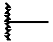 may mean that A₁ is covalently bound to Z at this position and 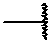 may mean that A₁ is covalently bound to A₂ at this position, or to A₃ if b is 0, or to L₂ if b and c are 0, or to E₂ if b and c are 0 and L₂ is a bond. a is an integer from 2 to 15, preferably 2 to 10, and more preferably 3 to 5, and even more preferably 4 to 5. X₁ is 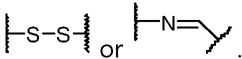 In the groups shown for X₁, 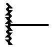 may mean that X₁ is covalently bound to Z at this position and 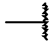 may mean that X₁ is covalently bound to the aromatic groups of A₁ at the shown position. A₂ is selected from the group consisting of 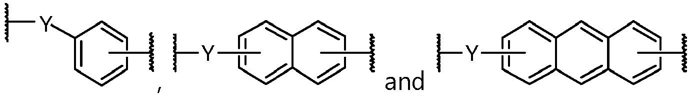 . In the groups shown for A₂, 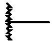 may mean that A₂ is covalently bound to A₁ at this position and 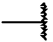 may mean that A₂ is covalently bound to A₃ at this position, or to L₂ if c is 0, or to E₂ if c is 0 and L₂ is a bond. Y is selected from the group consisting of a bond, especially a covalent bond, 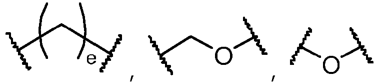 and 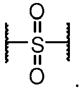 In the groups shown for X₁, 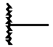 may mean that Y is covalently bound to A₁ at this position and 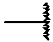 may mean that Y is covalently bound to the aromatic groups of A₂ at the shown position. e is an integer from 1 to 5, preferably from 1 to 2. b is an integer from 0 to 5, preferably from 0 to 3, and more preferably from 1 to 2. A₃ is selected from the group consisting of 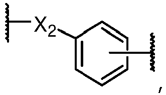 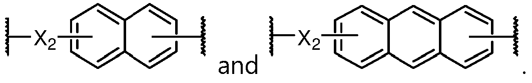 In the groups shown for A₃, 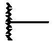 may mean that A₃ is covalently bound to A₂ at this position, or to A₁ if b is 0, and 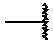 may mean that A₃ is covalently bound to L₂ at this position, or to E₂ if L₂ is a bond. X₂ is 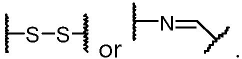 In the groups shown for X₂, 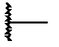 may mean that X₂ is covalently bound to A₂ at this position, or to A₁ if b is 0, and 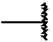 may mean that X₂ is covalently bound to the aromatic groups of A₃ at the shown position. c is an integer from 0 to 10, preferably 0 to 4, and more preferably from 1 to 2. Z is selected from the group consisting of 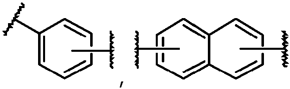 and 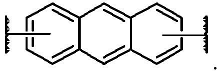 In the groups shown for Z, 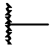 may mean that Z is covalently bound to L₁ at this position, or to E₁ if L₁ is a bond, and 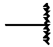 may mean that Z is covalently bound to A₁ at this position. L₁ is selected from the group consisting of a bond, especially a covalent bond, 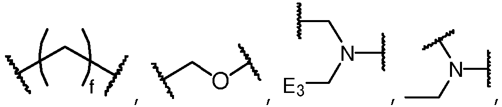 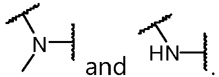 In the groups shown for L₁, 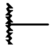 may mean that L₁ is covalently bound to E₁ at this position and 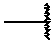 may mean that L₁ is covalently bound to Z at this position. f is an integer from 1 to 5, preferably from 1 to 2. E₃ is 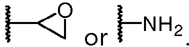 L₂ is selected from the group consisting of a bond, especially a covalent bond, 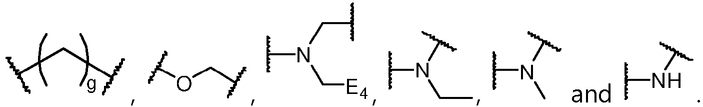 In the groups shown for L₂, 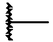 may mean that L₂ is covalently bound to A₃ at this position, or to A₂ if c is 0, or to A₁ if b and c are 0, and 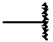 may mean that L₂ is covalently bound to E₂ at this position. g is an integer from 1 to 5, preferably from 1 to 2. E₄ is 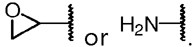 E₁ and E₂ are independently selected from the group consisting of 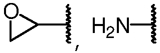 and H, preferably from the group consisting of 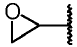 and 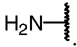 d is an integer from 1 to 5, preferably from 1 to 3, and more preferably, d is 1 or 2.

The inventors surprisingly found that uncured mixtures comprising or consisting of compounds according to Formula (I) exhibit increased viscosity, which enables tailoring the tack of the mixture. Furthermore, such mixtures as well as vitrimers formed using the compounds according to the first aspect of the invention have an enhanced performance in cryogenic environments. In addition, semi-finished products such as prepregs prepared using the compounds according to the present invention exhibit improved stiffness. The vitrimers formed using said compounds further exhibit an improved elasticity, toughness and intrinsic backbone flexibility. Without being bound to any particular theory, it is assumed that the specific structure of the compounds according to Formula (I) in connection with the chain length of the aromatic backbone are considered to be the reason for these favorable properties. In particular, the advantageous effects are considered to be ensured by the presence of at least two or more repeating units of A₁, optionally in combination with at least one further unit of A₂ and/or A₃. Furthermore, the compounds according to the invention enable the vitrimer material to stay high temperature resistant while having high modulus, low oxidation, low shrinkage and low water uptake by maintaining a closely cross-linked network.

In some embodiments, the aromatic and/or aliphatic groups of the compounds according to Formula (I) are optionally substituted with C₁ to C₆ alkyl groups, preferably C₁ to C₃ alkyl groups and/or C₆ to C₂₀ aryl groups, preferably C₆ to C₁₀ aryl groups. According to preferred embodiments, the aromatic groups of the compounds according to Formula (I) are optionally substituted with C₁ to C₃ alkyl groups. This has a beneficial effect on the viscosity of the monomers or monomer mixtures.

In some embodiments, the compound is a monomer and in particular a monomer for the formation and/or the manufacture and/or the production of a polymeric network and/or a vitrimer. According to certain embodiments, the compound is a chain-extender and in particular a chain-extender for the formation and/or the manufacture and/or the production of a polymeric network and/or a vitrimer.

According to certain embodiments, A₁ is selected from the group consisting of 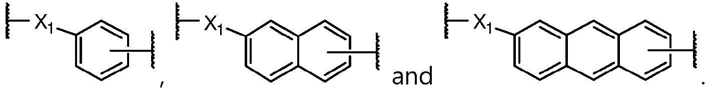 In some embodiments, A₁ is selected from the group consisting of 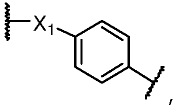 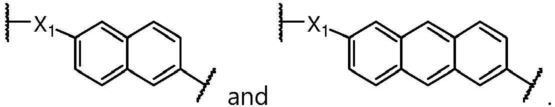 According to preferred embodiments, A₁ is 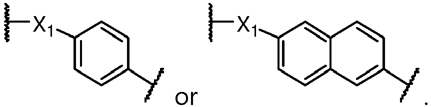 In these embodiments, the above-mentioned advantages and effects of the invention are particularly pronounced.

According to certain embodiments, A₂ is selected from the group consisting of 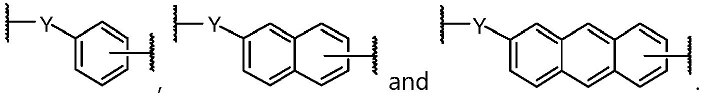 In some embodiments, A₂ is selected from the group consisting of 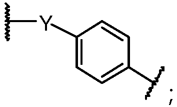 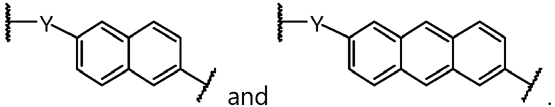 According to preferred embodiments, A₂ is 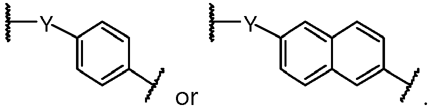 In these embodiments, the above-mentioned advantages and effects of the invention are particularly pronounced.

In some embodiments, A₃ is selected from the group consisting of 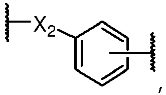 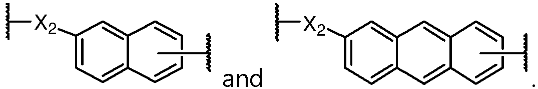 According to certain embodiments, A₃ is selected from the group consisting of 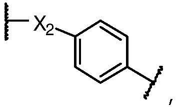 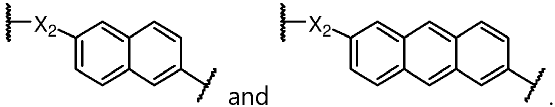 According to preferred embodiments, A₃ is 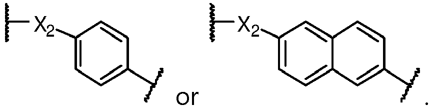 In these embodiments, the above-mentioned advantages and effects of the invention are particularly pronounced.

According to some embodiments, A₁ is selected from the group consisting of 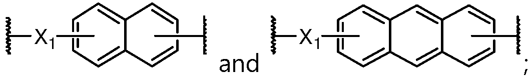 or from the group consisting of 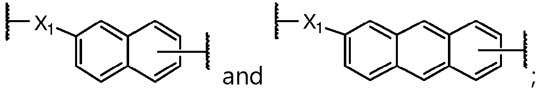 or from the group consisting of 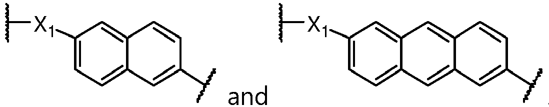 Alternatively or in addition, A₂ may be selected from the group consisting of 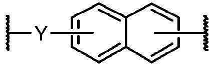 and 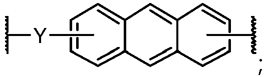 or from the group consisting of 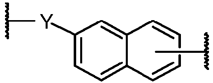 and 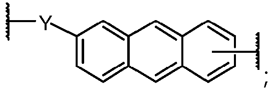 or from the group consisting of 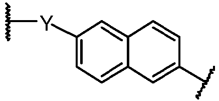 and 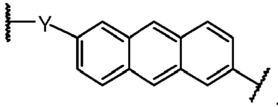 Alternatively or in addition, A₃ may be selected from the group consisting of 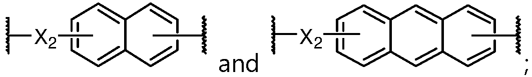 or from the group consisting of 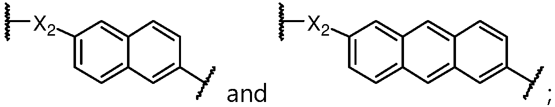 or from the group consisting of 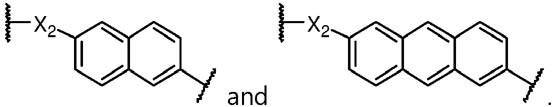 The inventors found that vitrimers formed using the compounds according to these embodiments have a further improved strength, stiffness and glass transition temperature (Tg), in addition to the advantageous effects stated above. Without being bound to any particular theory, it is assumed that the introduction of multiple rings such as double or triple rings in the compounds further increases the rigidity of the aromatic backbone, which leads to a further increase in the Tg.

In some embodiments, Z is selected from the group consisting of 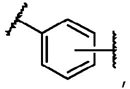 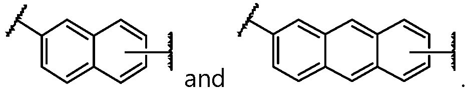 According to certain embodiments, Z is selected from the group consisting of 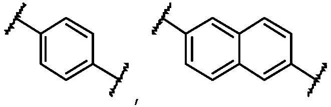 and 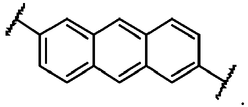 According to preferred embodiments, Z is 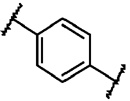 or 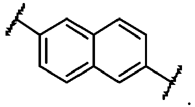 In these embodiments, the above-mentioned advantages and effects of the invention are particularly pronounced.

In certain embodiments, L₁ is 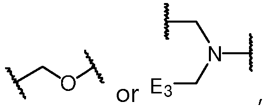 and preferably 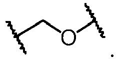 Alternatively or in addition, L₂ may be 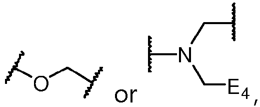 and preferably 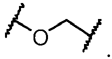 According to certain embodiments, X₁ is 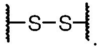 Alternatively or in addition, X₂ may be 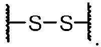 In some embodiments, Y is 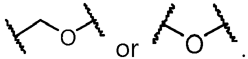 In these embodiments, the above-mentioned advantages and effects of the invention are particularly pronounced.

According to certain embodiments, the compound according Formula (I) contains either amine or epoxy groups. In other words, in some embodiments, the compound according Formula (I) does not have both amine and epoxy groups. In some embodiments, E₁ is 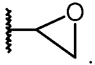 Alternatively or in addition, E₂ may be 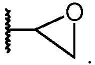 Alternatively or in addition, E₃ may be 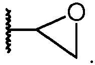 In preferred embodiments, E₁, E₂ and E₄ are 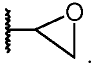 In these embodiments, the above-mentioned advantages and effects of the invention are particularly pronounced.

In certain embodiments, a is an integer from 2 to 4, b is an integer from 0 to 2, c is an integer from 0 to 2 and d is 1 or 2. According to preferred embodiments, a is 2 or 3, b is 1 or 2, c is 1 or 2 and d is 1 or 2. In these embodiments, the above-mentioned advantages and effects of the invention are particularly pronounced.

According to some embodiments, the compound is selected from the group consisting of 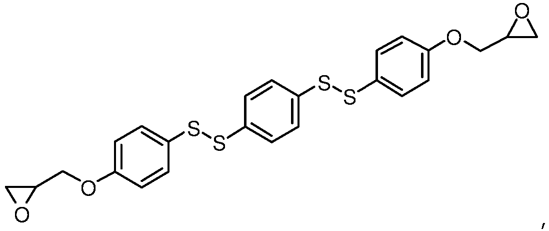 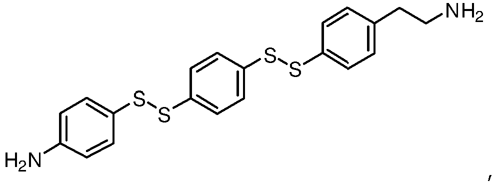 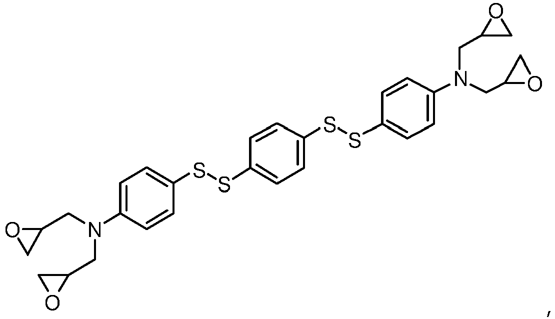 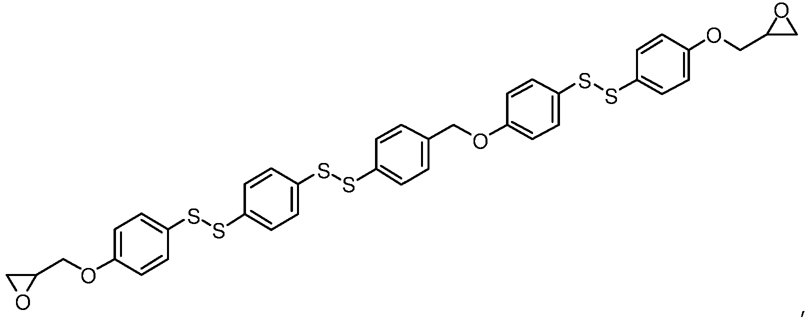 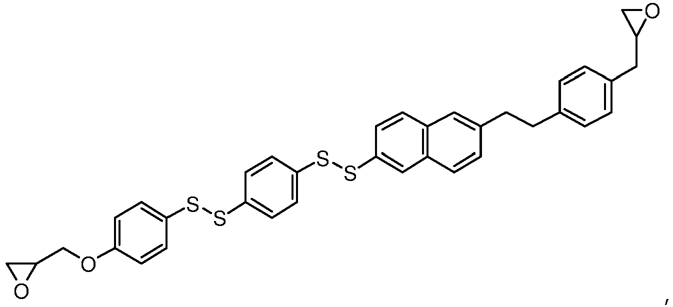 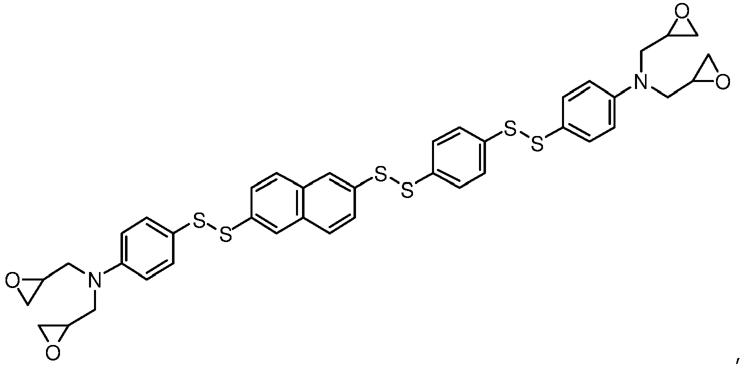 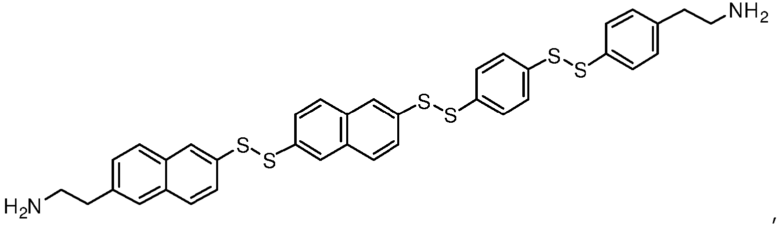 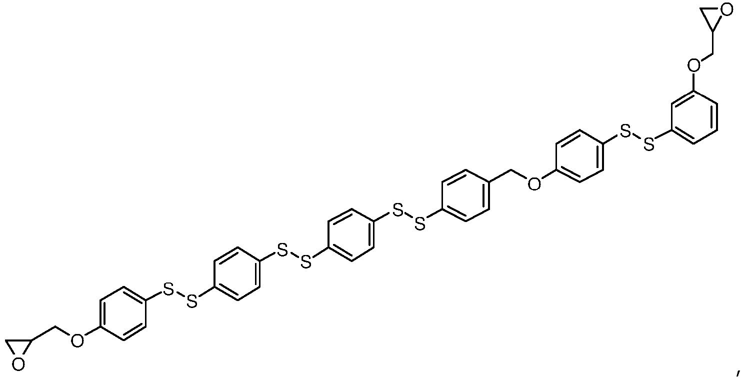 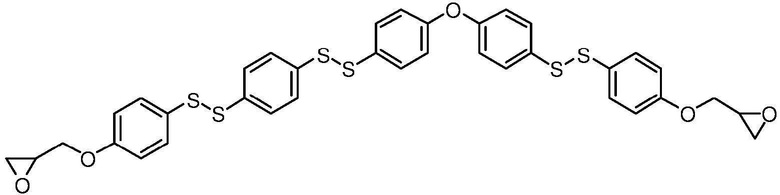 and 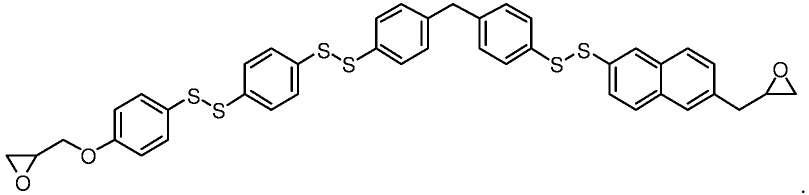 In some embodiments, the compound is 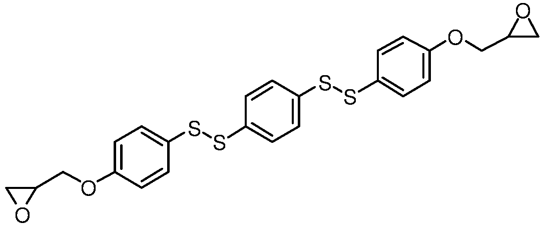 or 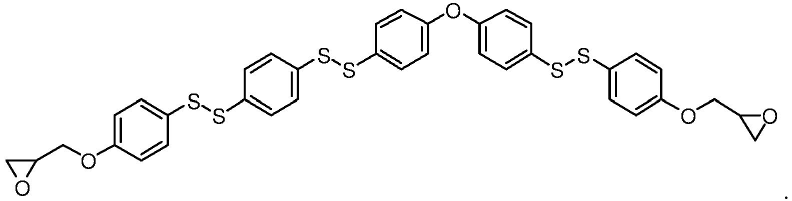 In these embodiments, the above-mentioned advantages and effects of the invention are particularly pronounced.

The compounds of the present invention can be polymerized and/or cross-linked in order to form and/or produce a polymeric network and/or a vitrimer and/or a composite material. In some embodiments, the compounds comprise epoxy groups. The compounds may be polymerized by the epoxy groups present in the compounds, for example by cationic polymerization. Alternatively or in addition, the compounds comprising epoxy groups may be cross-linked, for example by cross-linking agents and/or hardeners comprising amine, hydroxyl and/or thiol groups. According to certain embodiments, a first compound according to the invention comprises at least one amine group and a second compound according to the invention comprises at least one epoxy group. The first compound is different from the second compound. The first and second compound may be polymerized by the epoxy and amine groups present in the compounds, for example by polyaddition and/or step-growth polymerization. Alternatively or in addition, the first and second compounds comprising epoxy and amine groups may be cross-linked, for example by cross-linking agents and/or hardeners comprising amine, epoxy, hydroxyl and/or thiol groups. According to some embodiments, the compounds comprise disulfide groups. The compounds may be cross-linked, for example by cross-linking agents and/or hardeners comprising thiol groups, or by disulfide-disulfide exchange reactions.

Alternatively or in addition, the compounds according to the present invention can be used, for example, together with vitrimer monomers in order to form and/or produce a polymeric network and/or a vitrimer and/or a composite material. The vitrimer monomers may be selected from vitrimer monomers known in the art. In this context, "vitrimer monomers" refer to compounds different from the compounds according to the present invention and may be selected from the group consisting of 1,2-bis(4-(oxiran-2-ylmethoxy)phenyl)disulfane, bis-(4-aminophenyl)-disulfide, bis-(2-aminophenyl)-disulfid, bis-(3-aminophenyl)-disulfide, cystamine, diglycidyl ethyl disulfide, bis(gylcidoxy naphtyl) disulfide. The vitrimer monomers can be copolymerized with at least one compound of the present invention. Alternatively or in addition, the vitrimer monomers may be prepolymerized to form a prepolymer and the prepolymer subsequently reacted with at least one compound of the present invention to obtain a polymeric network and/or a vitrimer.

In a second aspect, the present invention relates to a composition comprising one or more compounds according to the first aspect of the present invention. Reference is made to the above statements on the first aspect of the invention, which apply analogously here. As mentioned above, such compositions and uncured mixtures exhibit increased viscosity and bulk stiffness, which enables tailoring the tack of the composition. Furthermore, such compositions as well as vitrimers formed using the compositions according to the second aspect of the invention have an enhanced performance in cryogenic environments. According to certain embodiments, the composition is an uncured mixture and/or a prepreg.

The composition can be applied to a substrate and then cured. Curing can be carried out, for example, by temperature treatment and/or the addition of a crosslinking agent and/or an initiator and/or a catalyst.

The composition may comprise or consist of two or more compounds according to the first aspect of the present invention. In some embodiments, the composition comprises or consists of a first compound according to the first aspect of the present invention and a second compound according to the first aspect of the present invention, wherein the first and second compounds are different from each other. In certain embodiments, the composition further comprises a cross-linking agent and/or a hardener. The cross-linking agent and/or hardener may be selected from the group consisting of 1,3,5-benzene trithiol (BTT), 1,4-benzene dithiol (BDT), 4,4'-biphenyl dithiol (BPDT), 2-thioaminophenol, 3-thioaminophenol, 4-thioaminophenol, 2,2'-dithiodianiline, 4,4'-dithiodianiline and 2-Amino-4-mercapto-6-methyl-1,3,5-triazine. In some embodiments, the composition comprises or consists of two or more compounds according to the first aspect of the present invention and the cross-linking agent and/or hardener. In these embodiments, the above-mentioned advantages and effects of the invention are particularly pronounced.

The composition may further comprise vitrimer monomers. The vitrimer monomers may be selected from vitrimer monomers known in the art. In this context, "vitrimer monomers" refer to compounds different from the compounds according to the first aspect of the present invention and may be selected from the group consisting of 1,2-bis(4-(oxiran-2-ylmethoxy)phenyl)disulfane and bis-(4-aminophenyl)-disulfide, bis-(2-aminophenyl)-disulfid, bis-(3-aminophenyl)-disulfide, cystamine, diglycidyl ethyl disulfide, bis(gylcidoxy naphtyl) disulfide. According to some embodiments, the composition comprises or consists of at least one compound according to the first aspect of the present invention, at least one vitrimer monomer and optionally at least one cross-linking agent and/or hardener. In these embodiments, the above-mentioned advantages and effects of the invention are particularly pronounced.

In a third aspect, the present invention relates to a method of manufacturing a polymeric network and/or a vitrimer. The method may comprise the steps of providing at least one compound according to the first aspect of the invention 1 and polymerizing, co-polymerizing and/or cross-linking the compound to obtain an oligomer and/or a polymer and/or the polymeric network and/or the vitrimer 2. Alternatively or in addition, the method may comprise the steps of providing the composition according to the second aspect of the invention 1 and polymerizing, co-polymerizing and/or cross-linking the composition to obtain an oligomer and/or a polymer and/or the polymeric network and/or the vitrimer 2. Reference is made to the above statements on the first and second aspects of the invention, which apply analogously here. According to some embodiments, the method is a method of manufacturing a vitrimer and comprises the steps of providing at least one compound according to the first aspect of the invention 1 and polymerizing and/or cross-linking the at least one compound to obtain the vitrimer 2. In certain embodiments, the method is a method of manufacturing a vitrimer and comprises the steps of providing the composition according to the second aspect of the invention 1 and polymerizing and/or cross-linking the composition to obtain the vitrimer 2.

The polymerization of the compound and/or composition is not particularly limited. According to certain embodiments, the polymerization is selected from the group consisting of cationic polymerization, polyaddition and step-growth polymerization, in particular from the group consisting of polyaddition and step-growth polymerization.

In some embodiments, the at least one compound comprises epoxy groups and the compounds are polymerized via cationic polymerization.

Alternatively or in addition, a first and second compound is provided, or the composition comprises a first and second compound, and the first compound may comprise at least one epoxy group and the second compound may comprise at least one amine group. Such compounds may be polymerized via step-growth polymerization.

Alternatively or in addition, the polymerization and/or the cross-linking may be conducted by the addition of a cross-linking agent and/or a hardener. In this context, reference is made to the above statements on the first aspect of the invention, which apply analogously here. The cross-linking agent and/or hardener may be selected from the group consisting of 1,3,5-benzene trithiol (BTT), 1,4-benzene dithiol (BDT), 4,4'-biphenyl dithiol (BPDT), 2-thioaminophenol, 3-thioaminophenol, 4-thioaminophenol, 2,2'-dithiodianiline, 4,4'-dithiodianiline and 2-Amino-4-mercapto-6-methyl-1,3,5-triazine.

In a fourth aspect, the present invention relates to a method of manufacturing a composite, comprising the steps of providing a composition according to the second aspect of the present invention, applying the composition to a substrate and curing and/or polymerizing and/or cross-linking the composition to obtain the composite. Reference is made to the above statements on the first, second and third aspects of the invention, which apply analogously here. In some embodiments, the substrate comprises or consists of a material selected from the group consisting of a thermoplastic, a thermoset, a metal and a ceramic.

In a fifth aspect, the present invention relates to a polymeric network or vitrimer or composite, produced using one or more of the compounds according to the first aspect of the invention and/or the composition according to the second aspect of the invention. In some embodiments, the polymeric network or vitrimer is produced by the method according to the third aspect of the invention. In some embodiments the composite is produced by the method according to the fourth aspect of the invention. According to certain embodiments, the polymeric network or vitrimer is produced by polymerizing and/or cross-linking one or more of the compounds according to the first aspect of the invention. In some embodiments, the polymeric network or vitrimer is produced by curing and/or polymerizing and/or cross-linking the composition according to the second aspect of the invention. Reference is made to the above statements on the first, second, third and fourth aspects of the invention, which apply analogously here.

In a sixth aspect, the present invention relates to a polymeric network or vitrimer or composite, obtainable and/or obtained from one or more of the compounds according to the first aspect of the invention and/or from the composition according to the second aspect of the invention. In some embodiments, the polymeric network or vitrimer is obtainable and/or obtained through the method according to the third aspect of the invention. In some embodiments the composite is obtainable and/or obtained through the method according to the fourth aspect of the invention. According to certain embodiments, the polymeric network or vitrimer is obtainable and/or obtained through polymerizing and/or cross-linking one or more of the compounds according to the first aspect of the invention. In some embodiments, the polymeric network or vitrimer is obtainable and/or obtained through curing and/or polymerizing and/or cross-linking the composition according to the second aspect of the invention. Reference is made to the above statements on the first, second, third, fourth and fifth aspects of the invention, which apply analogously here.

In a seventh aspect, the present invention relates to a composite, comprising a substrate and at least one vitrimer covalently bound to the substrate. According to certain embodiments, the at least one vitrimer comprises or consists of at least one compound according to the first aspect of the invention. In some embodiments, the at least one vitrimer is a vitrimer according to the fifth or sixth aspect of the invention. In some embodiments, the substrate comprises or consists of a material selected from the group consisting of a thermoplastic, a thermoset, a metal and a ceramic. The substrate may be in the form of a core or a layer. In some embodiments, the material is activated before forming the covalent bond, e.g. by thermal activation, irradiation, etc. This may especially be the case if the material is a metal or ceramic. The activation may be conducted to provide functional groups for forming the covalent bond. According to certain embodiments, the substrate comprises or consists of a thermoplastic or a thermoset, or mixtures thereof, preferably a thermoset. In some embodiments, the covalent bond between the vitrimer and the substrate is formed by reaction of epoxy, amine and/or disulfide groups of the vitrimer and functional groups of the substrate or material of the substrate. The functional groups may be selected from the groups consisting of hydroxyl, epoxy, amine and thiol groups. Reference is made to the above statements on the first, second, third, fourth, fifth and sixth aspects of the invention, which apply analogously here.

In an eighth aspect, the present invention relates to the use of a vitrimer as adhesive, matrix resin and/or for surface functionalization and/or the use of a composition according to the second aspect of the invention as adhesive, matrix resin and/or for surface functionalization. According to certain embodiments, the vitrimer comprises or consists of at least one compound according to the first aspect of the invention. In some embodiments, the vitrimer is a vitrimer according to the fifth or sixth aspect of the invention. In preferred embodiments, the vitrimer is used as adhesive, matrix resin and/or for surface functionalization in composites and especially in aircraft composites. The favorable performance in cryogenic environments as well as the tailorable elasticity and intrinsic backbone flexibility of the vitrimers and compositions of the present invention, as described above, makes them particularly suitable for aircraft applications. Reference is made to the above statements on the first, second, third, fourth, fifth, sixth and seventh aspects of the invention, which apply analogously here.

The above embodiments can be combined arbitrarily, if appropriate. Further possible embodiments and implementations of the invention comprise also combinations of features not explicitly mentioned in the foregoing or in the following with regard to the Examples of the invention. Particularly, a person skilled in the art will also add individual aspects as improvements or additions to the respective basic form of the invention.

### Examples

The present invention will now be described in detail with reference to several examples thereof. However, these examples are illustrative and do not limit the scope of the invention.

### Exemplary preparation of compounds according to the invention

The compounds according to the present invention can be prepared by means of syntheses customary in the art and familiar to the person skilled in the art. Exemplary methods of synthesis are shown below, but are not limited thereto.

### Preparation Example 1

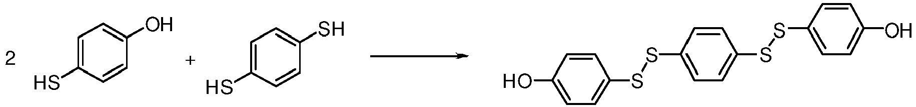

4-mercaptophenol (CAS 637-89-8) and 1,4-bezene dithiol (CAS 624-39-5) are commercially available. The disulfide formation can be carried out analogously to M. Pi 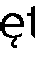 ta et al., Polym. Chem. (2023), 14, 7-31.

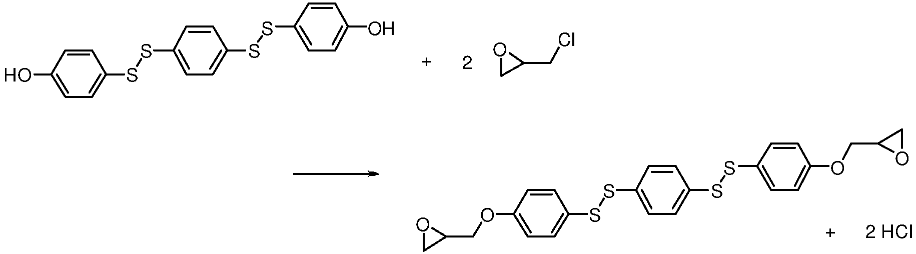

Epichlorohydrine (CAS 106-89-8) is commercially available.

### Preparation Example 2

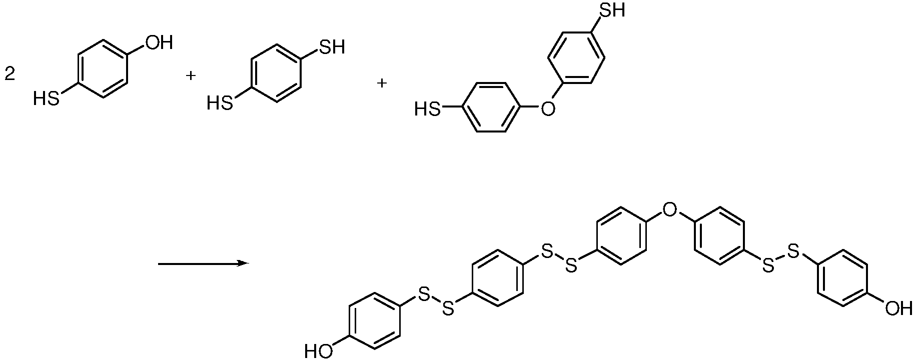

4-mercaptophenol (CAS 637-89-8), 1,4-bezene dithiol (CAS 624-39-5) and 4,4'-oxy(bisbenzenethiol) (CAS 17527-79-6) are commercially available. The disulfide formation can be carried out analogously to M. Pi 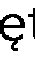 ta et al., Polym. Chem. (2023), 14, 7-31.

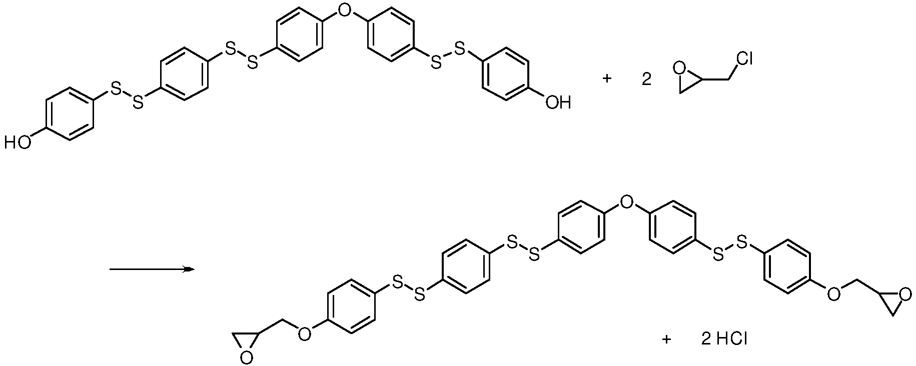

Epichlorohydrine (CAS 106-89-8) is commercially available.

### Preparation Example 3

The above syntheses of Preparation Examples 1 and 2 can also be carried out with Naphtalene-2,6-dithiol (CAS 96892-95-4) instead of 1,4-bezene dithiol, which is also commercially available.

### Exemplary preparation of vitrimers and composites according to the invention

The vitrimers and composites according to the present invention can be prepared by means of polymerization and cross-linking techniques customary in the art and familiar to the person skilled in the art. The compounds according to the present invention are polymerized and/or cross-linked and/or cured by methods as described above. For example, one or more of the compounds according to the invention are mixed, optionally with a cross-linker or hardener as described above, and the mixture is cured at temperatures between room temperature (22 °C) and 300 °C and in particular at temperatures between 80 °C and 200 °C. The compositions comprising the compounds according to the invention showed increased viscosity and bulk stiffness, which enabled tailoring the tack of the mixtures. Furthermore, the compositions as well as the vitrimers according to the invention exhibited enhanced performance in cryogenic environments. The vitrimers further showed an improved elasticity, toughness and intrinsic backbone flexibility.

### LIST OF REFERENCE SIGNS

- 1: providing at least one monomer according to the first aspect of the invention
- 2: polymerizing, co-polymerizing, cross-linking and/or curing the at least one monomer to obtain the polymeric network and/or vitrimer

## Claims

1. A compound according to the following Formula (I): 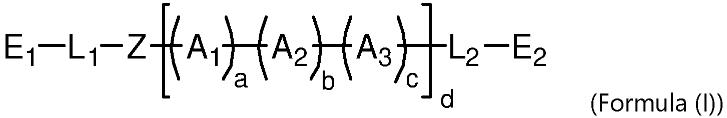 wherein A₁ is selected from the group consisting of 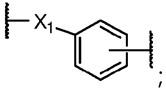 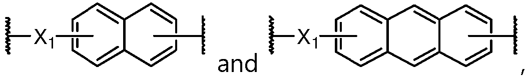 with a being an integer from 2 to 15, preferably from 2 to 10, and X₁ is 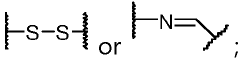 A₂ is selected from the group consisting of 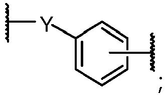 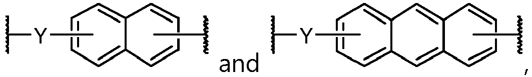 with b being an integer from 0 to 5, preferably from 0 to 3, and Y is selected from the group consisting of a bond; 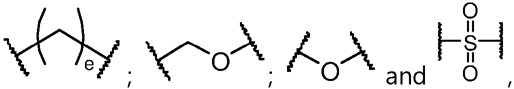 with e being an integer from 1 to 5, preferably from 1 to 2; A₃ is selected from the group consisting of 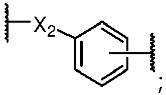 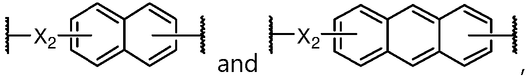 with c being an integer from 0 to 10, preferably from 0 to 4, and X₂ is 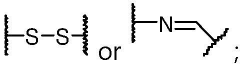 Z is selected from the group consisting of 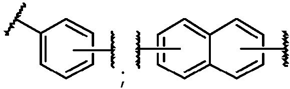 and 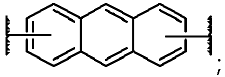 L₁ is selected from the group consisting of a bond; 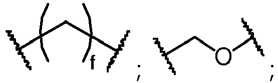 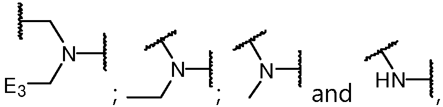 with f being an integer from 1 to 5, preferably from 1 to 2, and E₃ being 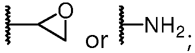 L₂ is selected from the group consisting of a bond; 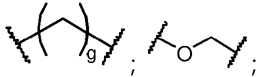 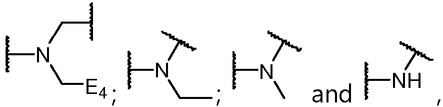 with g being an integer from 1 to 5, preferably from 1 to 2, and E₄ being 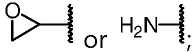 E₁ and E₂ are independently selected from the group consisting of 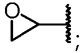 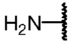 and H; and wherein d is an integer from 1 to 5, preferably from 1 to 3,
optionally wherein the aromatic and/or aliphatic groups of the compound according to Formula (I) are optionally substituted with C₁ to C₆ alkyl groups and/or C₆ to C₂₀ aryl groups.

2. The compound according to claim 1, wherein A₁ is selected from the group consisting of 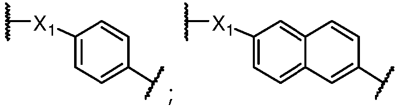 and 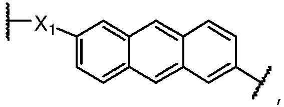 preferably wherein A₁ is 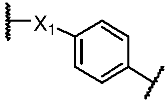 or 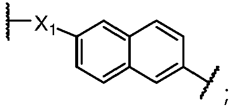 and/or wherein A₂ is selected from the group consisting of 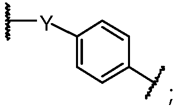 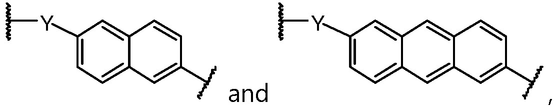 preferably wherein A₂ is 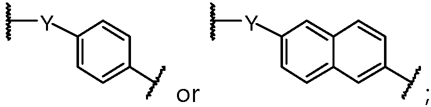 and/or wherein A₃ is selected from the group consisting of 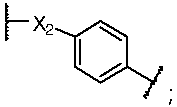 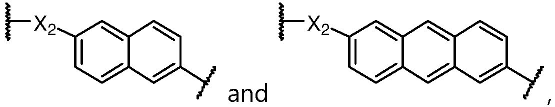 preferably wherein A₃ is

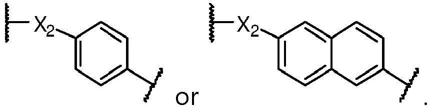

3. The compound according to claim 1 or 2, wherein Z is selected from the group consisting of 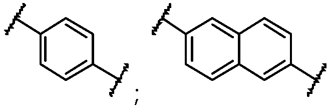 and 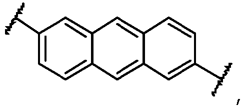 preferably wherein Z is 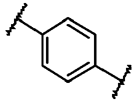 or

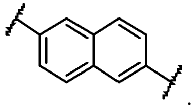

4. The compound according to any one of the preceding claims, wherein L₁ is 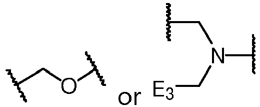 and/or L₂ is

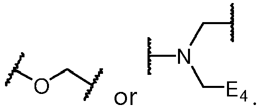

5. The compound according to any one of the preceding claims, wherein E₁, E₂, E₃ and/or E₄ is

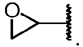

6. The compound according to any one of the preceding claims, wherein X₁ and/or X₂ are

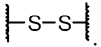

7. The compound according to any one of the preceding claims, wherein Y is

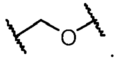

8. The compound according to any one of the preceding claims, wherein a is an integer from 2 to 4, b is an integer from 0 to 2, c is an integer from 0 to 2 and d is 1 or 2.

9. The compound according to any one of the preceding claims, wherein the compound is selected from the group consisting of 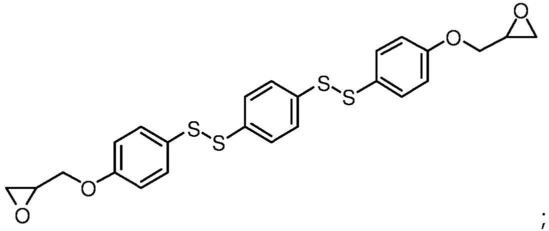 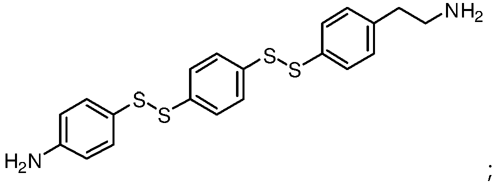 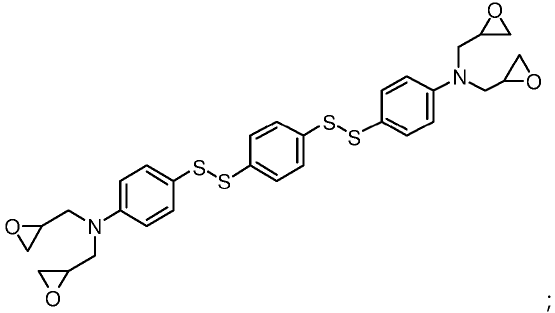 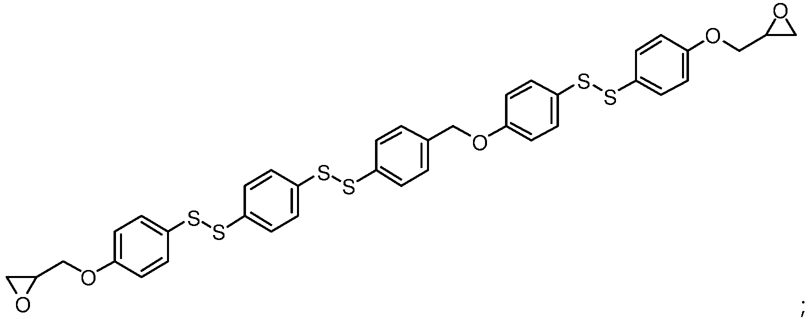 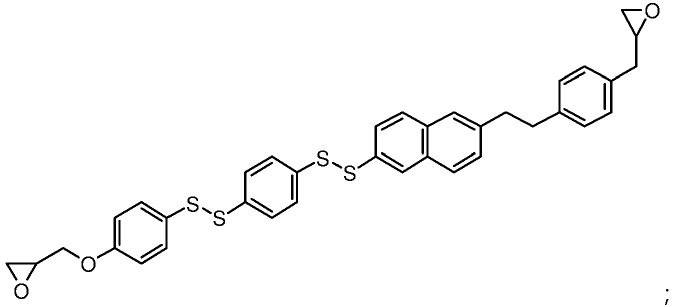 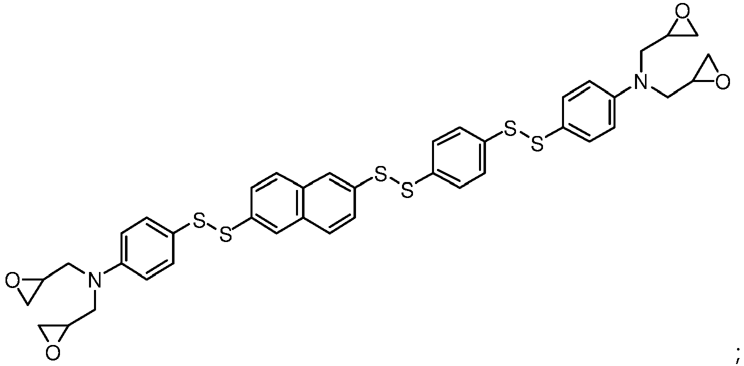 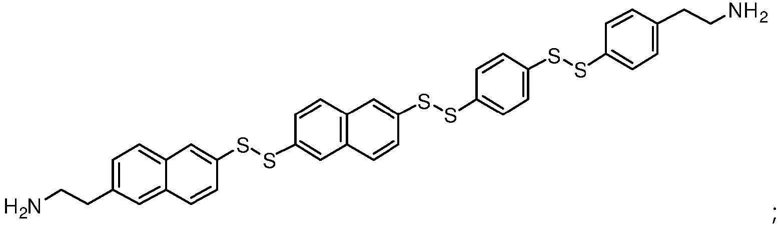 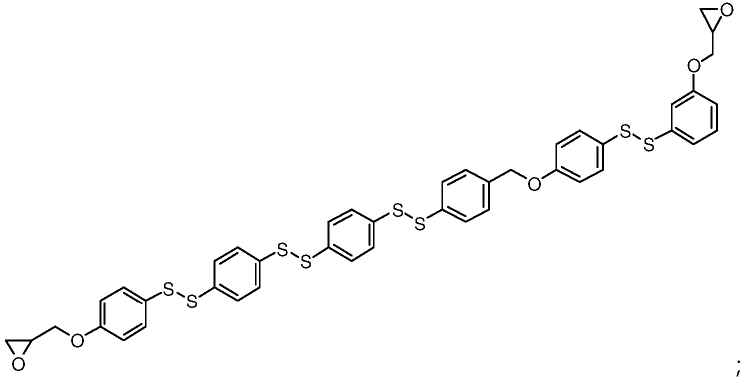 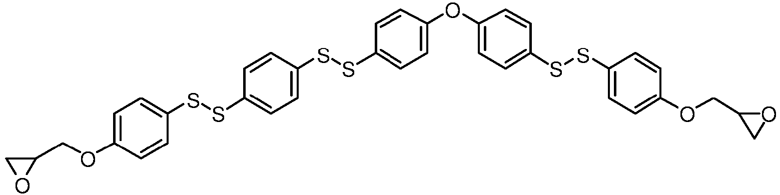 and

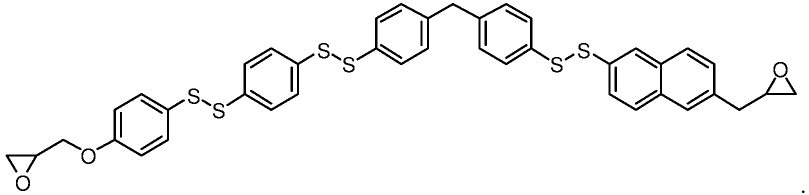

10. The compound according to any one of the preceding claims, wherein the compound is a monomer or chain-extender, in particular for the formation of a polymeric network and/or vitrimer.

11. A compositing, comprising one or more compounds according to any one of claims 1 to 10.

12. The composition according to claim 11, further comprising a cross-linking agent and/or a hardener.

13. A method of manufacturing a polymeric network and/or vitrimer, the method comprising:
providing at least one compound according to any one of claims 1 to 10 or
the composition according to any one of claim 11 or 12, polymerizing, co-polymerizing, cross-linking and/or curing the at least one compound or the composition to obtain the polymeric network and/or vitrimer.

14. A vitrimer or composite, produced using one or more of the compounds according to claims 1 to 10 and/or the composition according to claim 11 or 12.

15. Use of a vitrimer according to claim 14 or use of a composition according to claim 11 or 12 as adhesive, matrix resin or for surface functionalization, in particular in aircraft composites.
